# EUROPEAN PATENT APPLICATION

(11) **EP 1 977 734 A1**
(43) Date of publication of application: **08.10.2008**
(21) Application number: 07394008.2
(22) Date of filing: 03.04.2007
(51) Int. Cl.: A61K 9/00, A61K 9/14, A61K 9/20, A61K 9/28, A61K 31/196

(54) **A method of producing fast dissolving tablets**

(71) Applicant: Royal College of Surgeons in Ireland, Dublin 2 (IE)
(72) Inventor: The designation of the inventor has not yet been filed
(74) Representative: Purdy, Hugh Barry

(57) **Abstract**

A method of producing a fast-melt tablet comprises the steps of forming a mixture of components, the mixture comprising at least one fast dissolving sugar alcohol, at least one disintegrant or osmotic agent, and at least one an active component, blending the mixture for a period of time, and directly compressing the blended mixture at a compression force of typically between 5 and 20kN to form the fast-melt tablet. The process of the invention does not involve any granulation step, thereby making the process more energy efficient and cost effective. The fast dissolving sugar alcohol is selected from the group comprising: mannitol; sorbitol; erythritol; xylitol; lactose; dextrose; and sucrose, and comprises at least 50%, preferably at least 60%, and more preferably at least 70%, of the tablet (w/w). The active component is suitably provided in the form of microparticles or microcapsules having an average diameter of less than 125 microns. Also described are directly compressed fast dissolving type tablets obtainable by the process of the invention.

## Description

### Introduction

The invention relates to a method of producing fast dissolving tablets, and to fast dissolving tablets obtainable according to the method of the invention.

The use of conventional tablets is often challenging to geriatric, pediatric and uncooperative patients who have difficulties swallowing. Further, swallowing conventional tablets can be a problem when patients have a persistent cough or a gag-reflex, or when water is unavailable. These problems have been partly addressed by the provision of fast dissolving tablets in recent years. These tablet forms are also knows as FDDT (fast dissolving disintegration tablets), fast melt, or oral dissolving, tablets. Generally, these tablets include one or more hydrophilic disintegrants that, when placed on the tongue or in the oral cavity, rapidly absorb saliva and dissolve or disperse within less than one minute. A problem with the provision of these tablets is the need to provide a tablet that is sufficiently strong to withstand packaging, transport, and subsequent handling without breaking, yet capable of disintegrating rapidly when placed in the oral cavity. This problem has been addressed in a number of ways. Zydis (US Patents 4,305,502, 4,371,516 and 5738875) have addressed this issue by providing the tablet in the form of a friable freeze-dried matrix which, while dissolving rapidly in the mouth, is very fragile and requires the use of specialised packaging to ensure a minimum of handling of the tablet. Others have proposed the use of granulating, drying and tabletting technologies to provide a rapidly dispersible tablet that is more robust (EP0914818, WO2005/105049). While the tablets produced according to these process are less prone to breakage and, generally, do not require specialised packaging, the process involved in their manufacture is resource and energy intensive, and often requires the use of added functionality tablet excipients to enable processing of the materials.

It is an object of the invention to overcome at least one of the above-referenced problems.

### Statements of Invention

The invention provides a method of producing a fast dissolving type tablet which disintegrates rapidly in the mouth, which has acceptable characteristics of hardness and friability which obviate the need for specialised packaging. The method employs simple processing technology, including direct compression tabletting, and employs a relatively simple blend of excipients which allows for ease of processing. Surprisingly, it has been found that tablets produced according to the method of the invention have a high crushing strength, low friability of below 1% as per USP/BP method, and yet dissolve or melt rapidly in the mouth. Without being bound by theory, it is believed that the use of a fast dissolving sugar alcohol at a relatively high level, combined with the use of a disintegrant or osmotic agent, allows tablets be formed by direct compression resulting in a robust tablet that is capable of rapidly disintegrating in the oral cavity. It has further been found that the use of flat faced toolings provides a tablet of better disintegration characteristics than those formed with bi-convex toolings. Further, it is has been found that the incorporation of the active component into microparticles or microcapsules, typically formed by spray drying or spray chilling, enables the use of direct compression in the formation of a tablet having acceptable dissolution and hardness characteristics.

According to the invention, there is provided a method of producing a fast dissolving type tablet comprising the steps of forming a mixture of components, the mixture comprising at least one fast dissolving sugar alcohol, at least one disintegrant or osmotic agent, and at least one an active component, blending the mixture for a period of time, and directly compressing the blended mixture at a compression force of typically between 5 and 20kN to form the fast dissolving type tablet.

In a preferred embodiment, the process of the invention does not involve any granulation step, thereby making the process more energy efficient and cost effective. The process of the invention may employ pre-processed (and commercially available) components, such as, for example, the fast dissolving sugar Mannitol 200, Mannitol 300, Ludipress, Sorbitol 300, however the process of the invention does not involve granulation..

Suitably, the fast dissolving sugar alcohol is selected from the group comprising: mannitol; sorbitol; erythritol; xylitol; lactose; dextrose; and sucrose.. Preferably, the fast dissolving sugar alcohol is mannitol, ideally Mannitol 200. In one embodiment, the fast dissolving sugar alcohol comprises at least 50%, preferably at least 60%, and more preferably at least 70%, of the tablet (w/w). In one embodiment, the fast dissolving sugar alcohol comprises at least 85% of the tablet (w/w). In another embodiment, two different sugar alcohols are employed.

Typically, the disintegrant is selected from the group comprising: sodium starch glycolate (SSG); sodium carboxymethyl starch; calcium silicate; Cross linked N-vinyl-2-pyrrolidone; and crosscarmellose sodium, or combinations thereof. In one preferred embodiment, at least two disintegrants are employed such as, for example, EXPLOTAB and calcium silicate, or SSG and calcium silicate. Suitably, the disintegrant (or disintegrants) comprises between 5 and 40%, and preferably between 8 and 22%, of the tablet (w/w). Typically, the at least one disintegrant is a superdisintegrant. In another embodiment, the disintegrant is a superporous hydrogel. Suitably the superporous hydrogel is included at below 5% or less, preferably at 2% or less, and more preferably at about 1%. Examples of superporous hydrogels will be known to those skilled in the art.

Typically, the osmotic agent is selected from the group comprising anhydrous organic acids and salts thereof. In one embodiment, the osmotic agent is anhydrous citric acid or sodium citrate. Suitably, the osmotic agent (or agents) comprise between 5 and 15%, preferably between 8 and 12%, and more preferably between 9% and 11%, of the tablet (w/w).

In one preferred embodiment of the invention, the mixture of components additionally comprises a lubricant, typically selected from the group comprising: magnesium stearate; stearic acid, polyethylene glycol, polyoxyethylene- polyoxypropylene block copolymer (poloxamer). Suitably, the lubricant comprises between 0.1% and 5.0%, preferably between 0.2% and 1.0%, of the tablet (w/w).

In another embodiment, the lubricant, instead of or in addition to being included in the tablet formulation, is coated on to the faces of the tabletting dies. This is the method claimed by Eurand for their fast melt tablet

Optionally, the mixture of components includes a flow enhancing agent such as, for example, talc or colloidal silicon dioxide, at from 0.1% to 3.0%, and preferably from 0.1% and 0.5%, of the tablet (w/w). The mixture of components optionally includes a flavouring agent (such as, for example, synthetic oils, natural oils, or extracts from plants), typically at a level ranging from 0.5 to 4 % of the tablet (w/w). The mixture of components may also include a surfactant or wetting agent (such as sodium lauryl sulphate, Tweens, Spans), typically at a level of from 0.1 to 3% of the tablet (w/w).

The method of the invention involves the tablets being formed in a direct compression process. Suitably, a tablet press is employed. In a preferred embodiment, the tablet press has substantially flat faced toolings, ideally with a bevelled edge. Thus, the thickness of the formed tablet will not vary considerably from the centre to the edges (unlike tablets produced using bi-convex toolings which are thicker in the middle that at the edges). In a particularly preferred embodiment, the tablet has diameter in the range of 5-15mm, preferably in the range of 10-15mm and more preferably 15mm. Typically, the tablet has a diameter of at least 10mm, at least 11mm, at least 12mm, at least 12mm, and at least 14mm. Preferably, the tablet has a thickness of between 1 and 4 mm, preferably between 1.5-3.5 mm.

In a preferred embodiment of the invention, the compression force employed in the direct compression process is from 8kN to 20kN, typically from 10kN to 15kN.

Suitably, one or more of the components is provided in the form of microparticles having an average diameter of less than 125µ. In a preferable embodiment of the method of the invention, at least one active component is provided in the form of microparticles.
Typically, the microparticles have an average diameter of less that 125µ, preferably less than 100µ, preferably less than 50µ, preferably less than 20µ, preferably less than 10µ, preferably less than 5µ, preferably less than 4µ, preferably less than 3µ, preferably less than 2µ, preferably less than 1.5µ. In one embodiment, the microparticles have a mean diameter of about, or less than, 1.5µ. Generally, the microparticles are produced in a spray-drying or spray-chilling process.

In one embodiment, the microparticles have a solid or fluid core and a solid coating encapsulating the core (referred to hereafter as "microcapsules"). Such microcapsules may be formed in a process comprising the steps of providing a core-forming fluid stream and a coating-forming fluid stream, providing a two spray nozzle arrangement having a core nozzle disposed concentrically about a second nozzle, feeding the core-forming fluid stream to the core nozzle and the coat-forming fluid stream to the concentric nozzle to produce microcapsules, and solidifying the microcapsules immediately upon formation in a suitable gas.

Thus, the method of forming the microcapsules essentially comprises the steps of spraying a fluid stream through a nozzle to produce droplets, and drying (as in a spray drying process) or hardening (as in a spray chilling process) the droplets in air. Generally, the air will be hot air which dries the microcapsules as they leave the nozzle. However, in the case of spray chilling, in which case the fluid stream(s) comprise lipids and/or waxes and/or low melting point polymers, which are heated to melt these components, the microcapsules formed at the nozzle are solidified in cold air as opposed to hot air. General details of spray chilling methodology are available in the Quick Operation Guide

Generally, the method is characterised over conventional spray drying or spray chilling insofar as the nozzle comprises a core nozzle through which a core-forming fluid is sprayed and a second nozzle formed concentrically about the core nozzle and through which a coat-forming fluid stream is sprayed. The droplets formed by the double nozzle arrangement comprise a core of the first fluid and a coating of the second fluid.

In the case of spray drying, the hot gas is typically air or a different gas like an inert gas such as nitrogen, argon or other inert gases. In the case of spray chilling, air at ambient temperature of 45°C or below is generally used.

Typically, the core-forming fluid is a liquid or a gas. When it is of a liquid nature, it is selected from the group comprising: a solution; a suspension; a dispersion; a colloidal solution or dispersion; an oil; and an emulsion. Suitably, the core-forming liquid comprises an active compound or substance, optionally in combination with one or more pharmaceutically acceptable excipients. The active compound or substance may be any type of therapeutic, prophylactic, diagnostic, or prognostic agent. Further, it may be an agent used in imaging or labelling. In one preferred embodiment, the agent may be a pharmaceutically active agent that is required to be released in a controlled manner; thus, the coating may be designed to break down slowly in a physiological environment to release the encapsulated core over a period of time.

Typically, the core comprises a material/substance that is different to the material/substance of the coating.

Optionally the core may include a sustained release polymer with the coat being a second controlled release polymer with/without one or more targeting moieties.

In one embodiment, the core-forming fluid may comprise or consist of a gas or a volatile solvent such as but not limited to ethanol, acetone, ethylacetate. The gas may be selected from the group comprising: air; an inert gas; and a gas suitable for imaging applications. The use of a gaseous core finds particular application in microcapsules for pulmonary delivery, the gaseous core providing a microcapsule of low density more suited for delivery as an aerosol.

In one embodiment of the invention, the coat-forming fluid comprises a coating material capable of forming a film or wall around the core material. Suitably, the coat forming fluid comprises a component selected from the group comprising: polymer; lipid; wax; surfactants; surface stabilising agents; and ligands suitable for targeting the microcapsules to a specific desired site of action in the body. Suitably, the polymer is selected from the group comprising: methacrylate polymers such as Eudragit polymers; ethylcellulose polymers; biodegradable polyesters such as poly-lactide (PLA),polyglycolide (PGA),_and copolymers of lactic and glycolic acid, poly-lactide-co-glycolide (PLGA, poly-caprolactone (PCA); poly-amino acids; albumin; gelatine; alginate; and chitosan. Other suitable film-forming or wall-forming materials will be known to those skilled in the art.

The coat-forming fluid preferably comprises one or more agents selected from the group comprising: a pharmaceutically active agent; a taste masking agent (i.e. a sweetener); an agent that is liable to dissolution, swelling or degradation under certain defined (possibly physiological) conditions (a pH sensitive polymer, starch and starch derivatives, etc); a targeting compound (a ligand to a cell surface receptor overexpressed in tumour cells, i.e. vacuolar ATPases); an enhancer (short and medium chain fatty acids and their salts); a surfactant or wetting agent (tween, poloxamer, etc); and a surface stabilising agent (poloxamer, polyvinylpyrrolidone, etc).

In another embodiment, the coating may comprise a targeting moiety which is designed to target cells, tissues or organs to deliver the active agent. For example, the targeting moiety could be a ligand having a high affinity for a receptor that is highly expressed on the surface of tumour cells, i.e. ligands to vacuolar proton ATPases.

When spray chilling is employed, the coat-forming fluid may comprise lipids including phospholipids, waxes, surfactants or low melting point polymers which have a melting point of up to 75°C.

In one embodiment, the core nozzle has a diameter of between 0.7 and 2 mm. Typically, the concentric nozzle has a diameter of between 1.4 and 4 mm. Preferably, the core nozzle has a diameter of about 1mm and the concentric nozzle has a diameter of about 2mm. Alternatively, the core nozzle has a diameter of about 1.5mm and the concentric nozzle has a diameter of about 3mm. Alternatively, the core nozzle has a diameter of about 2mm and the concentric nozzle has a diameter of about 4mm. Generally, the diameter of the core nozzle is between 40% and 60%, preferably about 50%, the diameter of the concentric nozzle.

Suitably, the core and coat-forming fluid streams have a flow rate of up to 25ml/min depending on the viscosity of the solution and the pump setting.

The droplets formed by the nozzle are dried as they leave the nozzle and pass through the heated gas. As it is a spray drying process, the gas is hot air or a heated inert gas such as nitrogen, typically having an inlet temperature of between 80°C and 220°C (preferably between 90°C and 110°C, and ideally about 100°, when heated nitrogen is used). Suitably, the heated nitrogen has an outlet temperature of between 40°C and 70°C.

When heated air is used, the inlet temperature has a range 120-220°C and the outlet temperature between 60°C and 160°C.

The methods described above are suitable for forming microcapsules having a core encapsulated by a single coat. However, the method may be employed to produce microcapsules having two or more coats. Thus, the nozzle may comprise at least one further nozzle formed concentrically about the second nozzle and through which a further coat-forming fluid stream is sprayed. The use of multiple coats can have advantages in the sequential and controlled delivery of more than one active agent. Thus, for example, a microcapsule may be formed comprising a core containing a first active, a first coat comprising a second active, and an outer coat. In use, such a microcapsule would have a delayed release of the actives, with the second active being released first (but only after the outer coat is degraded), and the first active being delivered last. Alternatively, the components of the microcapsule could be chosen such that a sustained release of active is achieved through the provision of a number of different coats.

In a preferred embodiment of the method of the invention, the active is a highly potent pharmaceutical comprising less that 5%, preferably less that 4%, preferably less that 3%, preferably less that 2%, preferably less than 1%, preferably less than 0.5%, and preferably less than 0.2%, of the tablet (w/w). In such circumstances, the active is provided is the form of microparticles, or microcapsules, having a average diameter of less than 125µ, preferably less than 100µ, preferably less than 50µ, preferably less than 40µ, preferably less than 30µ, preferably less than 20µ, preferably less than 10µ, preferably less than 5µ, preferably less than 4µ, preferably less than 3µ, preferably less than 2µ, and preferably less than 1.5µ. The provision of the active in the form of a microparticle or microcapsule ensures a homogenous distribution of small particles of the active in the fast dissolving tablet, thereby increasing the bioavailability. Thus, the invention also relates to a method of producing a tablet of the type comprising a highly potent pharmaceutical active present in the tablet at less than 5%, preferably less that 4%, preferably less that 3%, preferably less that 2%, and preferably less than 1%, of the tablet (w/w), the method comprising the steps of producing a microcapsule or microparticle containing the highly potent active, blending the formed microparticles or microcapsules with other tablet excipients, and forming the tablet using suitable means. Typically, the tablet is formed by direct compression, however other tabletting means are also envisaged. Examples of such highly potent pharmaceutical actives include steroids and peptide therapeutics such as desmopressin. Other examples of highly potent actives that are used in small quantities will be well known to those skilled in the art.

The invention also relates to a directly compressed fast dissolving tablet comprising at least one fast dissolving sugar alcohol, at least one disintegrant or osmotic agent, and at least one an active component, and optionally a lubricant.

The invention also relates to a fast dissolving tablet consisting essentially of a fast dissolving sugar alcohol, between one and three disintegrants or osmotic agents, one active component, and a lubricant. Typically, the fast dissolving sugar alcohol is mannitol, preferably mannitol 200. Ideally, two disintegrants are employed, one of which is preferably EXPLOTAB. Suitably, a disintegrant is excluded, in which case an osmotic agent, such as anhydrous citric acid or sodium citrate, is employed.

The invention also relates to a directly compressed fast dissolving tablet consisting essentially of:
- 30% to 80% of a fast dissolving sugar alcohol;
- 5% to 40% disintegrant or osmotic agent;
- 0.1 % to 25% of active component;
- 0.1% to 1 % of lubricant.
the tablet having a disintegration time of less than 90 seconds and a hardness of greater than 30 Newtons. Preferably, the tablet has a disintegration time of less than 60 seconds, preferably less than 50 seconds, preferably less than 40 seconds, preferably less than 35 seconds, preferably less than 30 seconds, and preferably less than 25 seconds. Preferably, the tablet has a hardness of greater than 35 Newtons, preferably greater than 40 Newtons, preferably greater than 45 Newtons, preferably greater than 50 Newtons, preferably greater than 55 newtons, preferably greater than 60 Newtons, and preferably greater than 65 Newtons.

The invention also relates to a directly compressed fast dissolving tablet consisting essentially of:
- 50% to 80% of a fast dissolving sugar alcohol;
- 8% to 25% disintegrant or osmotic agent;
- 0.1 % to 25% of active component; and
- 0.1 % to 1% of lubricant,
the tablet having a disintegration time of less than 60 seconds and a hardness of greater than 40 Newtons.

Typically, the fast dissolving sugar alcohol is mannitol, preferably mannitol 200. Ideally, two disintegrants are employed, one of which is preferably EXPLOTAB. Suitably, a disintegrant is excluded, in which case an osmotic agent, such as anhydrous citric acid or sodium citrate, or superporous polyacrylic hydrogels or superabsorbant polymers such as Luquasorb® is employed. Preferably, the active is provided in the form of microparticles or microcapsules (as described above).

The invention also relates to a directly compressed fast dissolving tablet consisting essentially of:
- 50% to 80% of mannitol 200;
- 5% to 15% of EXPLOTAB and 5% to 15% of a further disintegrant; or
- 5% to 15% of an osmotic agent;
- 0.1 % to 25% of an active component; and
- 0.1 % to 1% of a lubricant,
wherein the tablet has a disintegration time of 60s or less, and has a hardness of at least 40kN.

Generally, the fast dissolving sugar alcohol is selected from the group comprising: mannitol; sorbitol; erythritol; xylitol; lactose; dextrose; and sucrose. Preferably, the fast dissolving sugar alcohol is mannitol, ideally Mannitol 200. In one embodiment, the fast dissolving sugar alcohol comprises at least 50%, preferably at least 60%, and more preferably at least 70%, of the tablet (w/w). In one embodiment, the fast dissolving sugar alcohol comprises at least 80% of the tablet (w/w). In another embodiment, two different sugar alcohols are employed.

Typically, the disintegrant is selected from the group comprising: sodium starch glycolate (SSG); sodium carboxymethyl starch; calcium silicate; Cross linked N-vinyl-2-pyrrolidone; and crosscarmellose sodium, or combinations thereof. In one preferred embodiment, at least two disintegrants are employed such as, for example, EXPLOTAB and calcium silicate, or SSG and calcium silicate. Suitably, the disintegrant (or disintegrants) comprises between 5 and 40%, and preferably between 8 and 22%, of the tablet (w/w). Typically, the at least one disintegrant is a superdisintegrant. In another embodiment, the disintegrant is a superporous hydrogel. Suitably the superporous hydrogel is included at below 5% or less, preferably at 2% or less, and more preferably at about 1%. Examples of superporous hydrogels will be know to those skilled in the art.

Typically, the osmotic agent is selected from the group comprising anhydrous organic acids and salts thereof. In one embodiment, the osmotic agent is anhydrous citric acid or sodium citrate. Suitably, the osmotic agent (or agents) comprise between 5 and 15%, preferably between 8 and 12%, and more preferably between 9% and 11 %, of the tablet (w/w).

In one preferred embodiment of the invention, the mixture of components additionally comprises a lubricant, typically selected from the group comprising: magnesium stearate; stearic acid, polyethylene glycol, polyoxyethylene- polyoxypropylene block copolymer (poloxamers). Suitably, the lubricant comprises between 0.1% and 5.0%, preferably between 0.2% and 1.0%, of the tablet (w/w). In another embodiment, the lubricant, instead of or in addition to being included in the tablet formulation, is coated on to the faces of the tabletting dies.

Optionally, the mixture of components includes a flow enhancing agent such as, for example, talc or colloidal silicon dioxide, at from 0.1% to 3.0%, and preferably from 0.1% and 0.5%, of the tablet (w/w). The mixture of components optionally includes a flavouring agent (such as, for example, synthetic oils, natural oils, or extracts from plants), typically at a level ranging from 0.5 to 3 % of the tablet (w/w). The mixtue of components may also include a surfactant or wetting agent (such as sodium lauryl sulphate, Tweens, Spans), typically at a level of from 0.1 to 3% of the tablet (w/w).

In one embodiment, the mixture of components includes a permeability enhancer such as, for example, sodium glycocholate or sodium caprate designed to enhance the buccal and/ oral permeability and absorption of poorly permeable actives

In another embodiment, the mixture of component includes a surfactant or wetting agent such as for example, Sodium lauryl suphate or poloxamer designed to enhance the solubility and absorption of poorly soluble actives

Ideally, the tablet has substantially flat faces (where the ratio of the thickness of the tablet at its centre and at its edges is not greater that 12: 10), and preferably a bevelled edge. Typically, the tablet has a diameter of at least 5mm, preferably at least 10mm, preferably at least 12mm, preferably at least 13mm, preferably at least 14mm, and preferably at least 15mm. Ideally, the tablet has a thickness of from 1 to 4mm, preferably from 1.5 to 2.5mm. In one preferred embodiment of the invention, at least one of the components of the tablet is provided in the form of microparticles or microcapsules having an average dimension of 125µ or less. Typically, the active is provided in the form of microparticles or microcapsules having an average dimension of 125µ or less.

The invention also relates to directly compressed fast dissolving tablets obtainable by the process of the invention.

The tablets of, and obtainable according to the process of, the invention suitably have a disintegration time of less than 90s, 60s, 50s, 45s, 40s, 35s, 30s, 25s, 20s, 15s, or 10s.

The tablets of, and obtainable according to the process of, the invention suitably have a weight variation of less than 3%, preferably less than 2%, and most preferably less than 1%.

Preferably, the tablet of, and obtainable according to the process of, the invention have a hardness of greater than 35 Newtons, preferably greater than 40 Newtons, preferably greater than 45 Newtons, preferably greater than 50 Newtons, preferably greater than 55 newtons, preferably greater than 60 Newtons, and preferably greater than 65 Newtons.

Typically, the tablet of, and obtainable according to the process of, the invention have a friability of 0.3-,0.9 % w/w according to USP method.

The invention will be more clearly understood from the following description of some embodiments thereof, given by way of example only.

### Detailed Description of the Invention

The examples below provide a number of fast dissolving tablets formed according to the process of the invention. The characteristics of the tablets were determined as follows:
Disintegration time (PharmaTest Disintegration tester, PTFE Germany)
Hardness or Crushing strength (PharmaTest tablet hardness tester, PTB 411E, Germany)
Uniformity of weight (Sarorius)
Thickness (Digital caliper, Workzone UK)
Friability (USB/BP method)

### Example 1

The following tabletting excipients were weighed and blended for 5 minutes in a sealed plastic bag. 37.25g of Mannitol 200, 5g of Explotab, 5 g of calcium silicate and 2.5 g of sodium diclofenac uncoated. After blending for 5 minutes, 0.25g of magnesium stearate was added and blended gently x 1 minute. The powder blend was then transferred to the hopper of a Piccola tablet press (An 8 station Rotary Tablet Press) fitted with 15 mm flat faced, bevelled edge round toolings and compressed at a force of 15 kN. Tablet were produced at a target tablet weight was 500mg. Tablets obtained were tested for weight uniformity, hardness and disintegration times. Tablets showed an average weight of 517mg, a hardness of 54newtons and a disintegration time of 1 minute and 20 seconds.

### Example 2

Example 1 was repeated using Eudragit E coated sodium diclofenac prepared by spray drying a solution of sodium diclofenac and Eudragit E in ethylacetate (as described below in Example 8). The formula used was adjusted to keep the content of diclofenac at 25mg/500mg tablet weight. 10g of Eudragit E coated sodium diclofenac was used instead of 2.5g of sodium diclofenac and was blended with 29.75g of Mannitol 200, 5g of Explotab and 5 g of calcium silicate. After 5 minute blending, 0.25g of magnesium stearate was added and blended gently x 1 minute. Tablets were produced at a compression force of 12 kN and showed a hardness of 72 Newtons and a disintegration time of 40 seconds. Average tablet weight was 420mg.

### Example 3

Placebo FDDTs were manufactured using a blend containing 44.75g of Mannitol 200, 5g of anhydrous citric acid and 0.25g of magnesium stearate. The blend was prepared as in Example 1 and tablets were produced at a compression force of 10 KN. Tablets produced had an average weight of 520mg and showed a hardness of 56 Newtons and a disintegration time of 16 seconds.

### Example 4

Example 3 was repeated using sodium citrate instead of anhydrous citric acid. The tablets produced had an average weight of 512mg and showed a hardness of 46 Newtons and a disintegration time of 9 seconds.

### Example 5

The following tabletting excipients were weighed and blended for 5 minutes in a sealed plastic bag. 39.75g of Mannitol 200, and 5g of SSG. After blending for 5 minutes, 0.25g of magnesium stearate was added and blended gently x 1 minute. The powder blend was then transferred to the hopper of a Piccola tablet press fitted with 15 mm flat faced, bevelled edge round toolings and compressed at a force of 15 kN. Tablets were produced at a target tablet weight was 500mg. Tablets obtained were tested for weight uniformity, hardness and disintegration times. Tablets showed an average weight of 551mg, a hardness of 37 Newtons and a disintegration time of 37 seconds.

### Example 6

Example 5 was repeated, but employing a compression force of 15 kN. Tablet were produced at a target tablet weight was 500mg. Tablets obtained were tested for weight uniformity, hardness and disintegration times. Tablets showed an average weight of 546mg, a hardness of 54 Newtons and a disintegration time of 37 seconds.

### Example 7

Example 5 was repeated, but employing a compression force of 20 kN. Tablet were produced at a target tablet weight was 500mg. Tablets obtained were tested for weight uniformity, hardness and disintegration times. Tablets showed an average weight of 541mg, a hardness of 97 Newtons and a disintegration time of 42 seconds.

### Example 8

A solution of sodium diclofenac and Ethylcellulose was prepared by dissolving 5.0 g of sodium diclofenac and 15.0 g of Ethylcellulose polymer in 200mls of ethanol using a magnetic stirrer. The solution was spray dried using the Bucchi 290 Laboratory spray drier to form microparticles. This was repeated twice and the microparticles from the 3 batches were blended. The average diameter of the blended microparticles was 8.42 ± 0.68 microns and the sodium diclofenac loading was at 24:80 (w/w). The sodium diclofenac microparticles were blended with mannitol, Kollidon CL-SF and chocolate flavouring at the following weight ratios of 20g of sodium diclofenac microparticles: 70.5g of Mannitol 200: 5 g Kollidon CL-SF: 4g Chocolate flavouring. 0.5 g of Magnesium stearate was then added to the blend. This blend was then tabletted using 15mm flat beveled edge tablet toolings at a compression force of 10kN. Tablets obtained had a weight uniformity of 515.92 ± 15.51 mg, a hardness of 39.01 ± 5.17 Newtons, a disintegration time of 32 ± 3 seconds, a friability of 0.58% and a sodium diclofenac content of 27.00 ±1.22 mg.

### Example 9

The following tabletting excipients were weighed and blended for 5 minutes in a sealed plastic bag. 93.9g of Mannitol 200, and 5g of Kollidon CL-SF and 0.6 g of raspberry flavouring. After blending for 5 minutes, 0.5g of magnesium stearate was added and blended gently x 1 minute. The powder blend was then transferred to the hopper of a Piccola tablet press fitted with 15 mm flat faced, bevelled edge round toolings and compressed at a force of 15 kN. Tablets were produced at a target tablet weight was 500mg. Tablets obtained were tested for weight uniformity, hardness and disintegration times. Tablets showed an average weight of 499 ± 15.51 mg, a hardness of 44.58 ± 2.98 Newtons and a disintegration time of 22 ± 2 seconds and a friability of 0.89%.

In this specification, the term "fast dissolving type tablets" should also be understood to include chewable tablets. Further, the tablets of, and obtainable by the process of, the invention find utility for both human and animal use, and for delivery of pharmaceutical, dietary, nutraceutical, and other forms of active components. Further, they may be provided in the form of tablets intended to be dissolved in a solution prior to ingestion, and also oral, vaginal and other routes of administration. The tablets of, and obtainable by the process of, the invention are also useful for the delivery of macromolecules, unpalatable actives, highly potent actives, and actives that are subject to first-pass metabolism. They are also useful for the sub-lingual delivery of actives.

The invention is not limited to the embodiment hereinbefore described which may be varied in both construction, detail and method steps without departing from the spirit of the invention.

## Claims

1. A method of producing a fast-melt tablet comprising the steps of forming a mixture of components, the mixture comprising at least one fast dissolving sugar alcohol, at least one disintegrant or osmotic agent, at least one lubricant, and at least one an active component, blending the mixture for a period of time, and directly compressing the blended mixture at a compression force of between 5 and 20kN to form the fast-melt tablet.

2. A method as claimed in Claim 1 which does not involve a granulation step.

3. A method as claimed in Claims 1 or 2 in which the active component is provided in the form of microparticles or microcapsules having an average diameter of less than 125µ.

4. A method as claimed in Claim 3 or 4 in the microparticles or microcapsules have an average dimension of less that 10µ.

5. A method as claimed in any preceding Claim in which the fast dissolving sugar alcohol is selected from the group comprising: mannitol; sorbitol; erythritol; xylitol; lactose; dextrose; and sucrose.

6. A method as claimed in Claim 5 in which the fast dissolving sugar alcohol comprises at least 60% of the tablet (w/w).

7. A method as claimed in Claim 6 in which the fast dissolving sugar alcohol comprises at least 80% of the tablet (w/w).

8. A method as claimed in any preceding Claim in which the mixture of components consists essentially of a fast dissolving sugar alcohol, at least one disintegrant or osmotic agent, a lubricant, and an active component.

9. A fast dissolving type tablet comprising a fast dissolving sugar alcohol, at least one disintegrant or osmotic agent, a lubricant, and an active component.

10. A fast dissolving type tablet as claimed in Claim 9 having a disintegration time of less than 60 seconds.

11. A fast dissolving type tablet as claimed in Claim 9 or 10 and having a hardness of at least 30 Newtons.

12. A directly compressed fast dissolving tablet consisting essentially of:
- 50% to 80% of a fast dissolving sugar alcohol;
- 8% to 25% disintegrant or osmotic agent;
- 0.1 % to 40% of active component;
0.1 % to 1% of lubricant, and optionally
- one or more of a flow enhancer, a flavouring agent, and a surfactant or wetting agent, the tablet having a disintegration time of less than 60 seconds and a hardness of greater than 30 Newtons.
